# EUROPEAN PATENT SPECIFICATION

(11) **EP 1 706 390 B1**
(45) Date of publication and mention of the grant of the patent: **19.05.2010**
(21) Application number: 05702217.0
(22) Date of filing: 12.01.2005
(51) Int. Cl.: C07D 261/00

(54) **ISOXAZOLE DERIVATIVES AND THEIR USE AS CYCLOOXYGENASE INHIBITORS**
ISOXAZOLDERIVATE UND DEREN VERWENDUNG ALS CYCLOOXYGENASEINHIBITOREN
DERIVES ISOXAZOLE ET UTILISATION EN TANT QU'INHIBITEURS DE CYCLOOXYGENASE

(30) Priority: 12.01.2004 IT MI20040019
(43) Date of publication of application: 04.10.2006
(73) Proprietor: Universita' degli Studi di Bari, 70121 Bari (IT); Universita' Degli Studi di Chieti, 66013 Chieti (IT)
(72) Inventor: SCILIMATI, Antonio c/o Dipt. Farmaco-Chimico, I-70125 Bari (IT); DI NUNNO, Leonardo c/o Dipt. Farmaco-Chimico, I-70125 Bari (IT); VITALE, Paola, I-70121 Bari (IT); PATRIGNANI, Paola, I-66013 Chieti (IT); TACCONELLI, Stefania, I-66013 Chieti (IT); PORRECA, Ettore, I-66013 Chieti (IT); STUPPIA, Liborio, I-66013 Chieti (IT)
(74) Representative: Trupiano, Federica
(86) International application number: PCT/IB2005/000047
(87) International publication number: WO 2005/068442

(56) References cited:
- EP-A- 0 026 928
- EP-A- 0 549 797
- EP-A- 0 633 254
- EP-A- 1 251 126
- WO-A-01/81332
- WO-A-96/25405
- WO-A-98/47880
- WO-A-02/083655
- WO-A-03/029230
- WO-A-03/031404
- WO-A-03/078408
- WO-A-03/087062
- WO-A-2004/017968
- WO-A-2004/037798
- WO-A-2005/007620
- DATABASE CA [Online] CHEMICAL ABSTRACTS SERVICE, COLUMBUS, OHIO, US; DAVIES, MARK W. ET AL: "A regioselective cycloaddition route to isoxazoleboronic esters" XP002335580 retrieved from STN Database accession no. 2001:603098 & CHEMICAL COMMUNICATIONS, vol. 17, 2001, pages 1558-1559,
- ROSTON, D. A. ET AL: "Comparison of drug substance impurity profiles generated with extended length columns during packed-column SFC" JOURNAL OF PHARMACEUTICAL AND BIOMEDICAL ANALYSIS, vol. 26, no. 3, 2001, pages 339-355, XP002335573
- TALLEY, JOHN J. ET AL: "4-[5-Methyl-3-phenylisoxazol-4-yl]- benzenesulfonamide, Valdecoxib: A Potent and Selective Inhibitor of COX-2" JOURNAL OF MEDICINAL CHEMISTRY, vol. 43, no. 5, 2000, pages 775-777, XP002335574
- TALLEY, JOHN J. ET AL: "N-[[(5-Methyl-3-phenylisoxazol-4-yl)- phenyl]sulfonyl]propanamide, sodium salt, parecoxib sodium: a potent and selective inhibitor of COX-2 for parenteral administration" JOURNAL OF MEDICINAL CHEMISTRY SCHEME 1, vol. 43, no. 9, 2000, pages 1661-1663, XP002335575
- DATABASE CA [Online] CHEMICAL ABSTRACTS SERVICE, COLUMBUS, OHIO, US; BELLEC, CHRISTIAN ET AL: "Preparation of trisubstituted isoxazoles by chemical and electrochemical reduction of .alpha.-acyl-.beta.-nitrostilbenes" XP002335581 retrieved from STN Database accession no. 1980:426329 & JOURNAL OF HETEROCYCLIC CHEMISTRY , 16(8), 1657-9 CODEN: JHTCAD; ISSN: 0022-152X, 1979,
- DI NUNNO L ET AL: "Regioselective synthesis and side-chain metallation and elaboration of 3-aryl-5-alkylisoxazoles" TETRAHEDRON, ELSEVIER SCIENCE PUBLISHERS, AMSTERDAM, NL, vol. 58, no. 13, 25 March 2002 (2002-03-25), pages 2659-2665, XP004344987 ISSN: 0040-4020
- DI NUNNO, LEONARDO ET AL: "Novel Synthesis of 3,4-Diarylisoxazole Analogues of Valdecoxib: Reversal Cyclooxygenase-2 Selectivity by Sulfonamide Group Removal" JOURNAL OF MEDICINAL CHEMISTRY, vol. 47, no. 20, 2004, pages 4881-4890, XP002335576

## Description

### BACKGROUND OF THE ART

The term "Non Steroidal Anti-Inflammatory Drugs" (NSAID) refers to a class of drugs that has been known for a long time and which is able to reduce inflammation, pain and fever. The term "non steroidal" distinguishes these drugs from corticosteroids with an anti-inflammatory activity, which are agents with a more marked anti-inflammatory activity, but which present significant and important side effects. The most known and used drugs belonging to the class of non-steroidal anti-inflammatory drugs (NSAID) are, for example, acetylsalicylic acid (*Aspirin*®) and the corresponding salicylates, ibuprofen (currently available on the market as *Advil*® and *Motrin*®)*,* naproxen (currently available on the market as *Naprelan*® and *A*/*eve*®) and indomethacin (currently available on the market as *Indocin*®)*.*

It has been ascertained that these drugs act by means of an action mechanism which blocks the synthesis of prostaglandin through the inhibition of the enzyme Cyclooxygenase (COX), known also as Prostaglandin G/H Synthase (PGHS). Through this action mechanism, the drugs belonging to the class of non-steroidal anti-inflammatory drugs (NSAID) show, alongside the anti-inflammatory action, negative side effects on the gastrointestinal apparatus, because prostaglandin synthesis is involved in both processes. For many years it was therefore believed that it was impossible to obtain non-steroidal anti-inflammatory drugs (NSAID) that were not characterised by significant side effects on the gastrointestinal system. Later, after having shown that the enzyme cyclooxygenase (COX), which catalyses the conversion of arachidonic acid to prostaglandin and thromboxane, exists in two isoforms, known as COX-1 and COX-2, which proved to be codified by two different genes, it was also ascertained that the form indicated as cyclooxygenase-1 (COX-1) is virtually expressed in a constitutive way in all tissues and is therefore indicated as "constitutive", while the form cyclooxygenase-2 (COX-2) is expressed in response to stimuli, for example of an inflammatory nature, and is indicated as "induced", even though many exceptions are known to the generalisation given above.

In particular, the "constitutive" form COX-1 is involved in the activity of the digestive tract, while the "induced" COX-2 isoform is involved in the inflammatory process.

Therefore it clearly appears the necessity to develop new drugs which selectively inhibit the "induced" form COX-2, involved in the inflammatory process, and which drugs are not inhibitors, or at least not selective inhibitors, of only the "constitutive" form COX-1, thus avoiding even serious adverse side effects on the gastrointestinal system. Selective COX-2 inhibitors therefore possess a significant pharmacological activity as non-steroidal anti-inflammatory drugs for the treatment of acute pain syndromes and chronic disorders of an inflammatory nature. For example, selective COX-2 inhibitor such as Rofecoxib, Celecoxib (currently available on the market with the name Celebrex®) and Valdecoxib (currently available on the market with the name Bextra®) are advantageously used for the treatment of rheumatoid arthritis, osteoarthritis, and for the treatment of acute pain associated, for example, with dental surgery and with primary dysmenorrhoea. Recently, Rofecoxib, trade name Vioxx®, was withdrawn from the market due to data deriving from a study carried out on patients treated, who showed an incidence of myocardial infarction in comparison with the non treated group. In fact, this product, while demonstrating a good anti-inflammatory activity and the absence of side effects on the gastrointestinal system, showed, on the basis of these initial studies, serious and significant side effects on the cardiovascular system. For this reason several studies have been undertaken to assess any side effects on the cardiovascular system of selective anti-inflammatory product COX-2 inhibitors.

It therefore appears clear that the use of derivatives known as COX-2 inhibitors involves a series of consistent and dangerous side effects in the cardiovascular field, with respect to what has been shown for the non-steroidal anti-inflammatory drugs that have been in use since a long time.

On the other hand, these compounds remain highly efficacious drugs for the treatment of inflammatory states, especially of medium and severe extent, and it is therefore important to interpret correctly the most significant side effects on the cardiovascular system that have recently been revealed.

Also during studies of cyclooxygenase inhibitors, a significant role of the constitutive form COX-1 was demonstrated in the development of carcinogenic pathologies, in particular in the development of Intestinal polyposis and in the onset of cutaneous and ovarian carcinomas. In parallel, it was also observed that COX-1 plays an important role in the onset, for example following surgery, of medium and strong pain. Moreover, it was seen that the administration of COX-1 inhibitors can be useful in the prevention and/or treatment of atherosclerosis. It is therefore important to select and identify specific cyclooxygenase inhibitors even of the "constitutive" COX-1 form.

WO2004/017968 discloses substituted isoxazole derivatives having an immunomodulatory action and/or inhibiting the release of cytokines, suitable for treating diseases associated with the immune system.

WO03/087062 discloses 4-(heterocyclyl)-benzenesulfoximine derivatives wherein the heterocycle ring may be i.a. an isoxazole, useful in the treatment of inflammatory diseases, especially wherein prostaglandins play a pathophysiological role.

WO02/083655 discloses i.a. aminosulfonyl benzene substituted isoxazol derivatives, useful for the treatment of inflammation and of other cyclooxygenase-2 mediated disorders.

WO01/81332 and EP1251126 disclose 2-fluorobenzenesulfonyl compounds wherein the benzene ring may be substituted with, i.a. an isoxazole, useful for treating cyclooxygenase-2 mediated disorders such as inflammation.

WO 03/029230 discloses a method for preparing benzenesulfonyl compounds, in particular a method for the preparation of 4-[5-methyl-3-phenylisoxazol-4-yl]benzenzsulfonamide (valdecoxib), which is useful in treating COX-2 related-disorders. Specifically cited compounds are the following; valdecoxib, parecoxib and parecoxib sodium.

WO 96/25405 discloses substituted isoxazole for the treatment of inflammation. All compounds of formula (III) are prepared by synthetic methods which are different from the process as claimed in the present invention, valdecoxib included also. 3,4-dipheny-5-methylisoxazole according to D10 is just a reaction intermediate and it is not isolated.

EP 0 633 254 A1 discloses Isoxazole derivatives represented by general formula (1) as inhibitors of lipoxygenase and cyclooxygenase are reported.

J. Med. Chem. 2000, 43, 775-7 reports the synthesis of Valdecoxib, 3,4-diphenyl-5-hydroxy-5-methylisoxazoline.

In J. Med. Chem. 2004, 47, 4881-4889 the synthesis and the pharmacological evaluation of a number of diarylisoxazoles is reported. In particular, it discloses the preparation of 3,4-diphenyl-5-methylisoxazole, 3,4-diphenyl-5-ethylisoxazole, 3-(5-chloro-2-furyl)-4-phenyl-5-methylisoxazole and valdecoxib by a novel synthetic methodology.

### OBJECTS OF THE INVENTION

The object of the present invention is to provide a class of compounds inhibitors of cyclooxygenase (COX-1 and/or COX-2), which show a pharmacological activity and which are therefore used in the treatment of inflammation, in the prevention and treatment of carcinomas, in particular intestinal, ovarian and cutaneous, and in the treatment of pain syndromes, in particular resulting from surgery. Another object of the present invention is to provide a process for the preparation of a class of compounds inhibitors of cyclooxygenase (COX-1 and/or COX-2), which is simple, which consists of a number of limited steps, and which is industrially applicable.

Yet another object of the present invention is to provide a pharmaceutical composition which comprises at least one cyclooxygenase inhibitor (COX-1 and/or COX-2), which is effectively used in the treatment of inflammation, in the prevention and treatment of carcinomas, in particular intestinal, ovarian and cutaneous, and in the treatment of pain, in particular resulting from surgery.

### DESCRIPTION OF THE INVENTION

These and yet other objects and respective advantages which will be better explained by the following description, are achieved by cyclooxygenase inhibitor compounds (COX-1 and/or COX-2), having the following general formula: 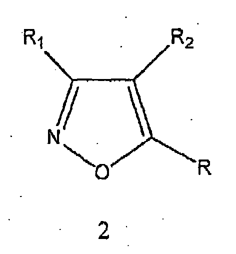 where:
R is a C₁₋C₅ alkyl linear; branched, not substituted;
R₁ is 5-chloro-2-furyl;
R₂ is phenyl.

Particularly, R is chosen as methyl (CH₃) or ethyl (CH₃CH₂). The derivatives with a general formula (2) according to the present invention, are generally prepared by reaction between arylnitryle oxides and free enolate ions (in turn obtained by metallation reaction of various alkyl methyl ketones with LDA in suitable conditions), followed by a dehydration/aromatization reaction and if necessary by further reactions of derivatization/modification of the derivatives thus obtained.

The present invention presents several advantages. As is better specified below, the new compounds according to the invention proved to be highly selective; the synthesis of this class of compounds according to the invention allows excellent yields to be obtained; the process of determining TLR4 polymorphism in subjects to be treated with anti-inflammatory drugs makes it possible to assess whether COX-2 inhibitors may or may not give rise to phenomena of significant toxicity affecting their cardiovascular apparatus. Moreover, that determination allows the assessment of the actual activity of the COX-1 and COX-2 inhibitor compounds.

The synthetic procedure for the preparation of 3,4-diaryl isoxazole derivatives, of general formula (2) is indicated in SCHEME 1 shown below:

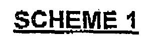

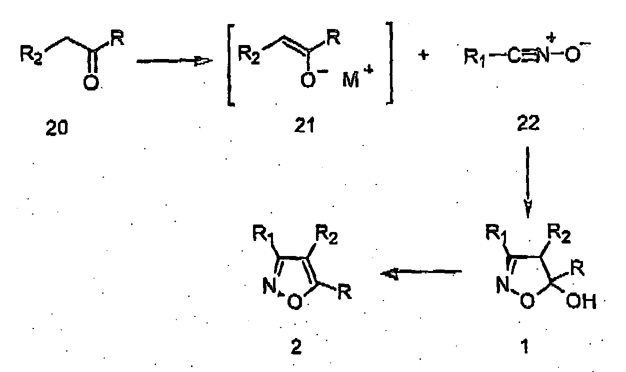

With reference to the scheme shown above, the key passage concerns the preparation of the desired enolate (21) from the corresponding ketone (20). Theoretically the ketone may lead to the formation of two different enolates in the presence of a base. According to the present invention, in the indicated synthetic step, conditions of precise thermodynamic control are used, in particular the reaction with LDA is carried out at the temperature of 0°C, so as to generate selectively the desired enolate. Latter, the enolate is reacted with various arylnitryle oxides (22) to give the 5-hydroxy-2-isoxazoline compounds (1). These compounds are obtained with good yields and with good diastereoisomeric ratios, as shown in Table 1.

**Table 1. Yields and diastereoisomeric ratios for the reaction between (21) and (22). R = methyl; R₂ = phenyl**

| Compound | R₁ | yield (%)^{a} | dr⁶ |
|---|---|---|---|
| **1c** | (c) 5-chloro-2-furyl | 45 | 80:20 |

| | | | |
|---|---|---|---|
| a) the yields refer to recrystallized products. b) the diastereoisomeric ratios were determined by means of ¹H NMR. | | | |

The diastereoisomeric ratios were determined by ¹H NMR spectroscopic analysis of the reaction crudes, in which the major isoxazoline isomer 1 (*cis*) has a more shielded δ_{CH3} than that of the minor isomer (*trans*)*,* as was ascertained in analogous systems. In fact, the chemical shift δ_{CH3} of the major isomer, for example the *cis* 1a, is closer (1.27 ppm) to that of an already known compound, *cis*-3,5-dimethyl-4-phenyl-5-hydroxy-2-isoxazoline (C), than it is (to the same already known compound) that of the minor isomer *trans.* On the contrary, the δ_{CH3} of the minor isomer (*trans* 1a) is closer to that of an already known compound *trans*-3-methyl-4-phenyl-5-hydroxy-2-isoxazoline (D). The effect of the phenyl group is more evident if one examines (B) where it is not present, and the chemical shift of methyl is 1.71 ppm, about the same as (D) in which methyl and phenyl are in *trans*.

The reaction between (22) when R₁ is chosen as (c) 5-chloro-2-furyl and enolate ions of ketones (21), also leads to the formation of appreciable quantities of corresponding isoxazole of formula (2), thanks to the basic conditions used during the reaction. All the above is simplified in **Table 2.**

**Table 2. yields of isoxazoline (1c) and isoxazole (2c) formed during the reaction between (22) when R₁ is chosen as (c) 5-chloro-2-furyl and enolate ion of ketone (21). R = methyl; R₂ = phenyl**

| R₁ | Reaction time | yield (%)^{a} | |
|---|---|---|---|
| | (h) | Isoxazoline | Isaxazole |
| 5-Chloro-2-furyl | | 45 (1c) | 13 (2c) |

| | | | |
|---|---|---|---|
| a) the Yields refer to products isolated by chromatography. | | | |

The 5-hydroxy isoxazoline compounds (1) according to the invention are then converted into the corresponding isoxazole compounds (2) in basic conditions. The yields and the reaction conditions are shown below in **Table 3.**

**Table 3. Yields of the isoxazole (2) obtained from the compounds with formula (1). R = methyl ; R₂ = phenyl**

| Compound | R₁ | Reaction time | yield (%)^{a} |
|---|---|---|---|
| 2c | - (c) 5-chloro-2-furyl | 1h | 60 |

| | | | |
|---|---|---|---|
| a) the yields refer to products isolated by chromatography. | | | |

The products to which the present invention refers, and in particular the compounds P6, proved to be excellent COX-1 inhibitors.

Given below are the chemical analyses of the above-mentioned compounds, obtained according to the general method described above.

### EXAMPLE 1

### General synthesis of the compounds 2

A solution of Na₂CO₃ (2.24 mmol) in water (10 mL) was added to 3-aryl-4-phenyl-5-hydroxy-6-methyl-2-isoxazoline (1.12 mmol) in THF (10 mL). The reaction mixture was then heated under reflux for the time indicated in table 3. The two phases were separated and the aqueous phase was extracted three times with ethyl acetate. The combined organic phases were dried on anhydrous Na₂SO₄ and the solvent was evaporated at reduced pressure to give the isoxazoles with a yield of 50-80%.
Analytical data of the product P6 (2c) **3-(5-Chloro-2-furyl)-4-phenyl-5-methylisoxazole (2c).**
60% yield, mp 71-73°C, yellow crystals. FT-IR (KBr): 3147, 3051, 2927, 2848, 1633, 1520, 1435, 1412, 1236, 1204, 1134, 1020, 985, 940, 926, 897, 796, 775, 704 cm⁻¹. ¹H NMR (CDCl₃, δ): 2.36 (s, 3H); 6.11-6.12 (d, 1H, J= 3.57 Hz); 6.25-6.27 (d, 1H, J= 3.57 Hz); 7.25-7.30 (m, 2H, aromatic protons); 7.40-7.47 (m, 3H, aromatic protons). ¹³C NMR (75 MHz, CDCl₃, δ): 11.41, 108.14, 113.87, 114.99, 128.58. 129.01, 129.63, 130.20, 138.59, 143.76, 152.42, 167.10. GC-MS (70 eV) *m*/*z* (rel.int.): 261 [M(³⁷Cl)⁺, 5], 259 [M(³⁵Cl)⁺, 15], 219 (11), 217 (36), 154 (17), 127 (10), 115 (5), 102 (5), 89 (14), 77 (9), 63 (10), 51 (12), 43 (100). Anal. calculated for C₁₄H₁₀NO₂Cl: C, 64.75; H, 3.88; N, 5.39. found: C, 64.73; H, 3.85; N, 5.38.

The products obtained according to the present invention were tested with an in vitro test on whole human blood isolated as described in Patrignani P. et al., J Pharmacol Exp Ther 1994; 271:1705-12*,* Patrignani P. et al., J Clin Invest 1982;69:1366-1372*;* Patrono C. et al., Low dose aspirin and inhibition of thromboxane B2 production in healthy subjects. Thromb. Res. 1980; 17: 317-27*.* The assays were carried out to assess the effects of the substituents and the activity of the compounds according to the present invention as inhibitors of COX-1/COX-2 activity, taking Valdecoxib (present trade name *Bextra*®) as a reference.

11 healthy volunteers (6 females aged between 25 and 29 years) were enrolled to participate in the study after each having given their written approval. The same volunteers had previously taken part in other studies.

### COX-2 Assay

1 ml aliquots of a sample of isolated peripheral venous blood samples containing 10 IU of sodium heparin were incubated in the laboratory in the presence of LPS (lipopolysaccharide) (10microg/ml) or saline for 24 hours at 37°C as described. The contribution of platelet COX-1 activity was suppressed by pre-treating the subjects with 300 mg of aspirin 48 hours before blood sampling. Plasma was separated by centrifugation (10 minutes at 2,000 r.p.m.) and kept at -70°C until assayed for prostaglandin (PG)E₂, as an index of LPS-induced monocyte COX-2 activity.

### COX-1 Assay.

Samples of isolated peripheral venous blood were used in vitro (taken from the same donors when they had not taken any non-steroidal anti-inflammatory drug during the two weeks preceding the study). 1 ml aliquots of isolated whole blood were immediately transferred into glass test tubes and kept at a temperature of 37°C for 1 hour. The serum was separated by centrifugation (10 min at 3000 rpm) and kept at -70°C until assayed for Thromboxane (TX)B₂. (TX)B₂ whole blood production was measured as a reflexion of the maximally stimulated platelet COX-1 activity in response to endogenous formed thrombin,

### Effect of the tested compounds on isolated whole blood COX-1 and COX-2 activities.

The compounds (0.005-150 mM) were dissolved in DMSO and aliquots of 2 microlitres of the solutions were pipetted directly into test tubes to give final concentrations of 0.01-300 microM in the blood. From 4 to 9 different concentrations of each compound were incubated with heparinised isolated whole blood samples in the presence of LPS (10 micrograms/ml) for 24 hours or with isolated whole blood samples allowed to clot at 37°C for 1 hour, in order to examine the concentration-dependence of COX-2 vs. COX-1 inhibition, respectively.

### Analysis of PGE₂ and TXB₂

The concentrations of PGE₂ and TXB₂ were measured by radioimmunological assays (Patrono C. et al., Low dose aspirin and inhibition of thromboxane B2 production in healthy subjects. Thromb. Res. 1980; 17: 317-27; Ciabattoni G. et al. J Endocrinol Invest 1979; 2:173-182). Isolated plasma and serum samples were diluted in the standard diluent of the assay (0.02M phosphate buffer, pH 7.4) and assayed in a volume of 1.5 ml at a final dilution of 1:50-1:30.000. 4000 d.p.m. of [³H]-PGE₂ or [³H]-TXB₂ were used and specific anti-PGE₂ and anti-TXB₂ antibody diluted 1:100,000 and 1:120,000, respectively. The least detectable concentration was 1-2 pg/ml for both prostanoids.

### Results

LPS-stimulated isolated whole blood samples, drawn from healthy subjects treated with aspirin 300 mg 48 h before sampling, produced 33±5.8 ng of

PGE₂ per ml of plasma (mean±S.E.M., n=13). TXB₂ production in clotting isolated whole blood samples, obtained from the same subjects in aspirin-free periods, averaged 466±53 ng/ml(mean±S.E.M., n=13).

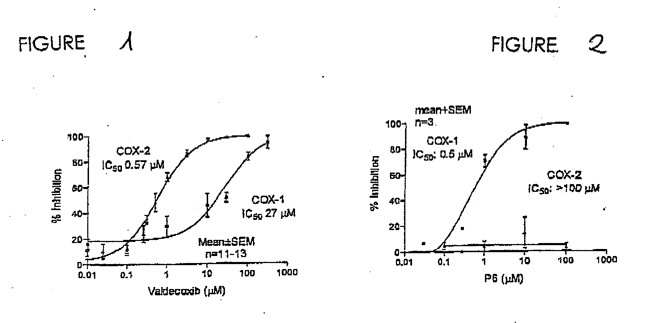

As shown in figures 1 and 2, the compounds P6 and the reference Valdecoxib inhibited LPS-induced monocyte COX-2 and thrombin-stimulated platelet COX-1 activities in a concentration-dependent fashion. IC₅₀ values for inhibition of platelet COX-1 and monocyte COX-2 activities are given in **Table 6.**

Valdecoxib inhibits monocyte COX-2 and platelet COX-1 activities with the following values of IC₅₀: 27 (16.22-44.63) and 0.57 (0.4619-0.7056) microM (95% interval).

The analysis of the sigmoidal concentration-response curves for inhibition of monocyte COX-2 and platelet COX by the compound P6, showed that virtually complete suppression (>90%) of platelet COX-1 activity occurs at concentrations that do not affect monocyte COX-2 activity (Figure 2).

**Table 6. Pharmacological data.**

| Compound | R₁ | | R | COX-1 IC₅₀(µM) | COX-2 IC₅₀(µM) |
|---|---|---|---|---|---|
| **22c(P6)** | 5-chloro-2-furyl- | phenyl | CH₃ | 0.5^{a} (0.2641-0.9248) | >100^{a} |
| **Valdecoxib** | phenyl | phenyl SO₂NH₂ | CH₃ | 27^{d} (16.22- 44.63) | -0.57^{a} (0.4619- 0.7056) |

| | | | | | |
|---|---|---|---|---|---|
| The values are means of at least two experiments ^{a}n=3 ^{d}n=11 ^{e}n=13 (values in parentheses are the IC₅₀ confidence intervals) | | | | | |

The inventors surprisingly found a significant role of the receptor TLR4 (Toll-like receptor **4**) in the onset and progression of toxicity affecting the cardiovascular system of subjects treated with COX-2 inhibitors.

Recently, epidemiological and experimental studies have revealed an important role of TLR4 in the progression and development of atherosclerosis (Cook et al. Nature Immunology 2004; 5:975-9). In particular, an association has been reported between the presence of the polymorphisms of TLR4 and a reduced atherosclerosis and also a lower risk of acute coronary events for the carriers of said polymorphism (Kiechl et al. New Engl J Med. 2002; 347:185-9; Edfelt et al. Eur Heart J 2004; 25: 1447-53; Ameziane et al 2003; 23: 61-4).

Thromboxane A₂ and prostacyclin which, as has already been said, represent the major products of the metabolism of arachidonic acid in the platelets and in the endothelial cells, perform a fundamental role in cardiovascular homeostasis. In particular, thromboxane A₂ causes platelet aggregation, vasoconstriction and vascular proliferation, while prostacyclin inhibits the platelet aggregation induced by many agonists, the vascular proliferation of the smooth muscle cells and vascular tone. Thus while thromboxane A₂ promotes the initiation and the progression of atherogenesis, prostacyclin prevents it. The side effects on the cardiovascular system, caused by the administration of cyclooxygenase-2 (COX-2) inhibitors, would therefore be due to the reduction of prostacyclin (which performs an athero-protective role), caused by the inhibition of COX-2 which participates in and contributes to the biosynthesis of prostacyclin in human. The enzyme COX-1, the principal isoform of COX responsible for the biosynthesis of thromboxane A₂ (which has a negative effect on the vascular system), not being inhibited by the anti-inflammatory compounds that selectively inhibit COX-2, does not undergo any variations and maintains the standard levels of thromboxane A₂. Thus, the balance between the athero-protective and anti-thrombotic effect of prostacyclin (the biosynthesis of which depends mainly on COX-2) and the "negative" effect of thromboxane A₂ (the biosynthesis of which depends mainly on COX-1) is altered by **COX-2 selective inhibitors** of in favour of thromboxane A₂, with consequent reduction of the "protective" effect of prostacyclin, and increase of damage to the cardiovascular system.

The inventors assessed the biosynthesis of prostacyclin and of thromboxane in subjects carriers of TLR4 polymorphism (Asp299Gly and Thr399IIe) in comparison with "wild-type" (WT) subjects who do not present said polymorphism, matched for age, sex and cardiovascular risks factors, in the absence of treatment with Aspirin®.

From the experimental results given below in detail, the following was surprisingly found.

In comparison with wild-type subjects, in subjects who were **carriers** of TLR4 polymorphisms, the urinary levels of 11-dehydro-TXB₂ and of 2,3-dinor-6-keto-PGF1α, systemic indices of thromboxane A₂ and of prostacyclin biosynthesis respectively, were significantly reduced. Moreover the ratio between the biosynthesis of prostacyclin and of thromboxane A₂ (prostacyclin/thromboxane ratio) was significantly higher in the carriers of TLR4 polymorphism than in wild-type subjects. Thus, in subjects who are carriers of TLR4 polymorphism, the reduced biosynthesis of platelet thromboxane seems to contribute to a greater cardiovascular protection and to reduced atherosclerosis, in comparison with wild-type subjects.

This consideration was used as the starting point for carrying out a study assessing the degree of potential toxicity of selective COX-2 inhibitors affecting the cardiovascular system.

Treatment with rofecoxib causes a similar inhibition of prostacyclin (about 60%) in the two groups of patients and is associated with an increased urinary excretion of 11-dehydro-TXB₂ in carriers of TLR4 polymorphisms, but not in wild-type subjects. When treated with selective COX-2 inhibitors, wild-type subjects undergo a reduction of the biosynthesis of prostacylin (COX-2 dependent) with a consequent reduction of the "protective" activity with respect to the cardiovascular system, typically carried out by prostacyclin as already said. Since they are selective COX-2 inhibitors, COX-1 platelet activity remains more or less unchanged, and so the level of thromboxane A₂ in vivo remains unchanged, and the consequent "negative" effect on the cardiovascular system. The result is a moderate onset of risk factors for the cardiovascular system, given by the decrease of prostacyclin, without a significant reduction of the levels of thromboxane A₂.

When treated with selective COX-2 inhibitors, carriers of TLR4 polymorphisms also undergo a reduction of the biosynthesis of prostacylin (COX-2 dependent), **but** they show a contemporary and surprising increase in the production of thromboxane A₂. This increase is so significant that the values of thromboxane A₂ may reach, and even exceed, the levels normally present in the wild-type population and so they are such as to bring back to at least standard conditions the carriers of TLR4 polymorphisms (who, as has already been said, in the absence of treatment, have lower levels of Thromboxane A₂ and therefore represent a "cardioprotected" phenotype with respect to WT subjects).

So the presence of TLR4 polymorphism seems to amplify dramatically the consequences of the inhibition of vascular prostacyclin by selective COX-2 inhibitors on platelet function.

In practice, in subjects who are carriers of TLR4 polymorphism treated with selective COX-2 inhibitors, the moderate effect of potential cardiovascular toxicity, which can be found in WT subjects for the reasons indicated above, is enormously amplified, thanks to the increase in the level of thromboxane A₂ with respect to what is observed in the absence of treatment with COX-2 inhibitors in the same carriers of TLR4 polymorphism, and therefore results in a potential cardiovascular toxicity. So, while selective COX-2 inhibitors could be administered to WT subjects with a moderate and at any rate acceptable risk of complications for the cardiovascular system, the administration of the same compounds to TLR4 polymorphism subjects would involve an extremely high and probably unacceptable risk for damages to the cardiovascular apparatus. So for these subjects characterised, in the absence of treatment with COX-2 inhibitors, by a reduced urinary excretion of 11-dehydro-TXB₂, the administration of selective COX-2 inhibitors would be unadvisable, but this would not be the case in WT subjects, for whom the beneficial effects of anti-inflammatory activity could be higher than the potential cardiovascular toxicity.

The results shown above were obtained on the basis of the following assessments.

### Subjects studied.

The study protocol was approved by the Ethical Committee of the University of Chieti "G.d'Annunzio", Faculty of Medicine.

The written approval of all the subjects studied was obtained.

408 outpatients were examined who took part in a programme for assessing cardiovascular risk at the Department of Internal Medicine.

The genotype of all for TLR4 polymorphism Asp299Gly and Thr399IIe was assessed, according to the method of Lorenz et al. (Biotechniques 2001; 31:22-4). 26 subjects proved to be carriers of TLR4 polymorphism.

In short, the DNA extracted from the peripheral blood was amplified with PCR using two specific pairs of primer for the regions containing Asp299Gly and Thr399IIe polymorphisms:
Asp299Gly, forward 5'-gattagcatacttagactactacctccatg-3'; reverse 5'-gatcaacttctgaaaaagcattcccac-3';
Thr399IIe, forward 5'ggttgctgttctcaaagtgattttgggagaa-3'; reverse 5'acctgaagactggagagtgagttaaatgct-

The two PCR products were digested using restricting enzymes Nco I and Hinf I, respectively. After digestion, the samples were made slide on 3% agar gel. For Asp299Gly polymorphism, the wild type allele was represented by a band of 249 bp, while the mutant allele was shown by a band of 226 bp. For Thr399IIe polimorfismo, the wild type allele was represented by a band of 406 bp, while the mutant allele was shown by a band of 377 bp.

An allele frequency of 2.8% was calculated and a carriage rate of 6.3%. Seven subjects were excluded from the study because they were under treatment with aspirin for cardio-protection. The 19 selected subjects were subdivided as follows: 9 heterozygous for Asp299Gly and Thr399IIe, 5 heterozygous for Asp299Gly and "wild-type" for Thr399IIe, 3 "wild-type" for Asp299Gly and heterozygous for Thr399IIe, 1 heterozygous for Thr399IIe and homozygous for Asp299Gly and 1 "wild-type" for Asp299Gly and homozygous for Thr399IIe. These 19 subjects were compared with 19 subjects who were not carriers of TLR4 polymorphism, **matched for age, sex and cardiovascular risk factors.** The characteristics of the subjects studied are listed below in Table 7.

**Table 7. Baseline characteristics of subjects who were carriers of TLR4 polymorphisms Asp299Gly and Thr399IIe and of wild-type subjects.**

| | "wild-type" *TLR4* | *TLR4 Polymorphisms* |
|---|---|---|
| No. | 19 | 19 |
| Age (years) (mean±DS) | 56±11 | 56±11 |
| sex, F(%) | 10 (53%) | 10 (53%) |
| Body mass index, (Kg/m²) (mean±DS) | 30±7 | 30±7 |
| hypertension, n°(%) | 14 (73) | 15 (79) |
| Diabetes, n°(%) | 2 (10) | 3 (16) |
| Smokers, n°(%) | 2(10) | 2 (10) |
| Cholesterol LDL, mg/dl | 135±47 | 123±27 |
| (mean±SD) | | |
| | | |
| **Drugs** | | |
| Nitrates, n°(%) | 0 (0) | 1 (5) |
| β-Blockers, n°(%) | 1(5) | 4 (21) |
| Calcium channel blockers, n°(%) | 4 (21) | 6 (32) |
| ACE inhibitors, | n°(%) 13 (68) | 9 (47) |
| Diuretics, n°(%) | 3 (16) | 5 (26) |
| Statins, n°(%) | 3(16) | 3 (16) |

All the subjects were studied by means of standard interviews, clinical examination and biochemical analyses. General biochemical assessments were carried out with traditional laboratory methods. Cholesterol LDL was calculated using the Friedewald formula. At the time of assessments, the subjects did not present any infection in progress or any inflammatory condition.

### Study protocols.

For the 19 carriers of TLR4 polymorphism and for the 19 control carriers of "wild-type" TLR4, overnight samples (from 8 p.m. to 8 a.m.) were collected to determine, on isolated samples, the levels of 11-dehydro thromboxane B₂, one of the principal enzymatic metabolizes of Thromboxane A₂. 11-dehydro-thromboxane B₂ represents an index of the systemic biosynthesis *"in vivo"* of Thromboxane A₂. The urinary levels of 2,3-dinor-6-keto-prostaglandinF_{1α} were also determined, which is one of the principal enzymatic metabolites of prostacyclin and represents an index of its systemic biosynthesis *"in vivo",* and the values of 8-iso-prostaglandin F_{2α}, index of oxidative stress "*in vivo".* Samples of whole blood were also collected and isolated to assess "*in vitro"* platelet COX-1 activity, as determined by the measurement of serum Thromboxane B₂, and of COX-2 monocyte activity, determined by the production of PGE₂ on whole blood stimulated with lipopolysaccharide *(Escherichia coli)* (10 µg/ml) for 24 hours, as reported in Patrignani et al., J Pharmacol Exp ther 1994;271:1705-12.

Six subjects who were carriers of TLR4 polymorphism and 6 "wild-type" TLR4 control subjects were treated with rofecoxib (a selective COX-2 inhibitor) (trade name VIOXX®) (25 mg a day, at 8 a.m.) for 5 consecutive days. Subjects with disturbances of a haemorrhagic nature, allergy to aspirin or to other non-steroidal anti-inflammatory drugs (NSAID) or with a history of gastrointestinal disorders were excluded from the study. The subjects refrained from using aspirin or other non-steroidal anti-inflammatory drugs (NSAID) for at least two weeks prior to the study. The blood samples were taken to determine COX-1 platelet activity and COX-2 monocyte activity before treatment with the drug and 12 hours after the last administration (at 8 a.m. on the sixth day of the study).

The collection of overnight urine samples was obtained to assess the urinary excretion of 11-dehydro-thromboxane B₂ and of 2,3-dinor-6-keto-PGF_{1α} before treatment with the drug and after the last dose of rofecoxib.

The study was completed before the voluntary withdrawal of Rofecoxib from the market by Merck due to the significant increase of the incidence of serious and significant side effects at cardiovascular level (Fitzgerald GA. N Engl J Med 2004;351:1709-11).

### Biochemical analyses.

The levels of TXB₂, PGE₂, 11-dehydro-TXB₂, 2,3-dinor-6-keto-PGF_{1α} and 8-iso-PGF_{2α} were measured with previously validated radioimmunological techniques (Patrono C, Ciabattoni G, Pinca E, et al. Low dose aspirin and inhibition of thromboxane B2 production in healthy subjects. Thromb Res 1980;17:317-27*.* Patrignani P, Panara MR, Greco A, et al. Biochemical and pharmacological characterization of the cyclooxygenase activity of human blood prostaglandin endoperoxide synthases. J Pharmacol Exp Ther 1994;271:1705-12*.* Ciabattoni G, Maclouf J, Catella F, et a/, Radioimmunoassay of 11-dehydrothromboxane B2 in human plasma and urine. Biochim Biophys Acta 1987;918:293-7*.* Wang Z, Ciabattoni G, Creminon C, et al. Immunological characterization of urinary 8-epiprostaglandin F2 alpha excretion in man. J Pharmacol Exp Ther 1995;275:94-100).

The levels of soluble CD40L, of the chemokine MCP-1 and of the vascular cellular adhesion molecule (VCAM-1) in citrated plasma were determined by ELISA determination - enzyme-linked immunosorbent assay (R & D System) according to the manufacturer's instructions. The concentration of fibrinogen in the plasma was determined by analysing its enzymatic conversion into fibrin. The C-reactive protein in the serum (CRP) was determined with the nephelometric method (Behring).

### Statistical analysis.

All the values are given as median and range, unless otherwise indicated.

The comparisons between the groups were made with the χ² test and the Mann-Whitney test. The correlations were determined with the Spearman rank-test. The statistical significance was indicated with *P<0.05.* In the pharmacological study, the primary hypothesis was that rofecoxib (25 mg/day) would cause a 60% reduction in the urinary excretion of 2,3-dinor-6-keto-PGF_{1α}. Assuming an inter-subject coefficient of variation of 30% for the urinary excretion of 2,3-dinor-6-keto-PGF_{1α}, the primary end-point, in carriers of TLR4 polymorphisms, 6 volunteers should have allowed a difference of 62% to be found in its measurement with a power of 90% based on a two-tailed test with values of P lower than an error factor of 0.05 of the first type. 6 TLR4 polymorphism carrier subjects and 6 non carriers were therefore enrolled. The software SPSS Inc (version 11.5 for Windows) was used for all the statistical analyses.

The results will now be commented with reference to the enclosed figures, where:

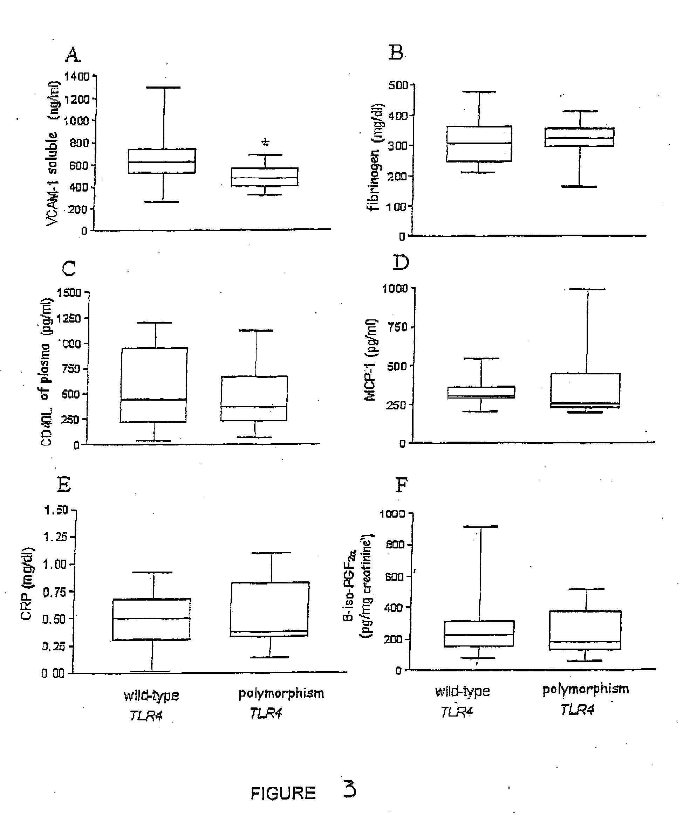

- fig. 3 shows the levels of biological markers of inflammation in carriers of *TLR4* polymorphism and in "wild-type" *TLR4* carriers. Levels of VCAM-1 (A), fibrinogen (B), CD40L (C), chemokine MCP-1 (D) and seric PCR (E) were assessed. The urinary excretion of 8-iso-prostaglandin F_{2α}, an index of oxidative stress *"in vivo",* was assessed in the collection of night urine (from 8 p.m. to 8 a.m.) (F). The data are reported as a mean and a confidence interval. [**P*=0.008 for the comparison between the carriers of *TLR4* polymorphism and of "wild-type" *TLR4*].

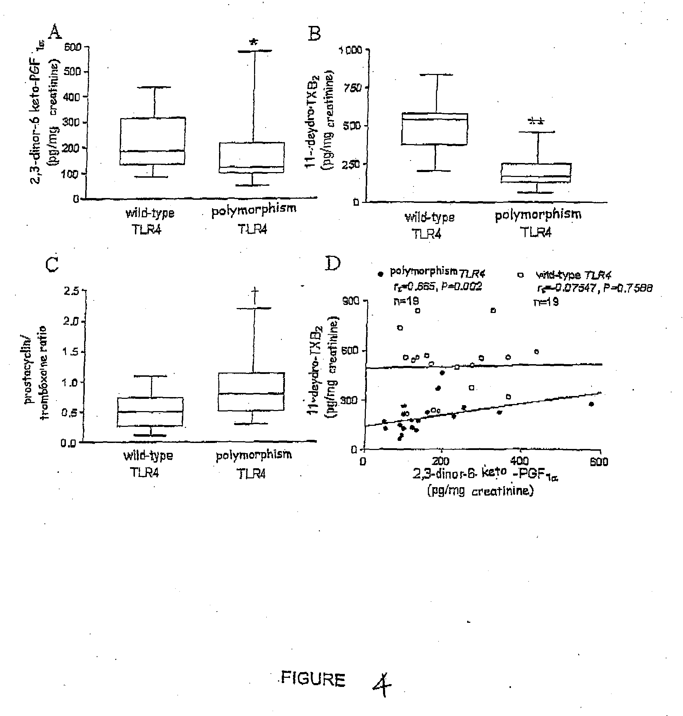

- fig. 4 refers to the biosynthesis of prostacyclin and thromboxane A2 in carriers of *TLR4* polymorphism and in "wild-type" *TLR4* carriers. Box-and-Whisker plot of urinary excretion of 2,3-dinor-6-keto-prostaglandin F_{1α} (A), one of the principal enzymatic metabolites of prostacyclin which is an index of its systematic biosynthesis *in vivo*, and 11-dehydro-thromboxane B₂ (B), one of the principal enzymatic metabolites of thromboxane A₂ which is an index of its systematic biosynthesis *in vivo,* and the prostacyclin/thromboxane A₂ ratio (C), in overnight urine collection (from 8 p.m. to 8 a.m.) of 19 subjects who were carriers of *TLR4* polymorphism and 19 "wild-type" controls. The data are reported as a mean and a confidence interval. (**P*=0.041, ***P*<0.0001, *P*=0.0061 for the comparison between the carriers of *TLR4* polymorphism and of "wild-type" *TLR4*). Correlation between 2,3-dinor-keto-prostaglandin F_{1α} and 11-dehydro-thromboxane B₂ in carriers and non carriers of *TLR4* polymorphism (D).

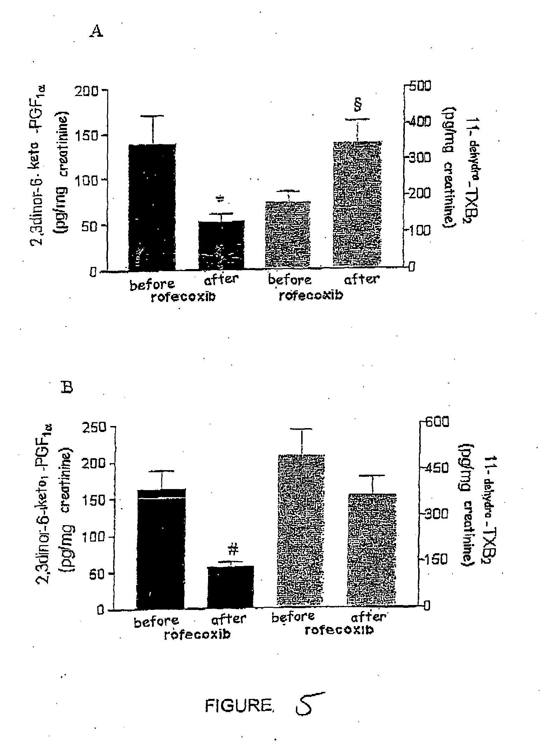

- fig. 5 shows the effects of rofecoxib (25mg per day for 5 consecutive days) on the biosynthesis *in vivo* of prostacyclin and thromboxane in carriers of *TLR4* polymorphism (A) and in carriers of "wild-type" *TLR4*(B). The data are reported as means and standard errors. [**P*= 0.0087, ^{§}*P*=0.041, #P=0.0043 with respect to the values prior to taking the drug (pre-drug)]. *Levels of inflammatory biomarkers.*

As shown in figure 5A-E, the circulating levels of plasma soluble VCAM-1, but not of fibrinogen, of MCP-1, CD40L and CRP, were significantly lower in carriers of TLR4 polymorphisms than in the wild-type [473 (324-681) *versus* 624 (260-1301) ng/ml, median (range), respectively, *P*=0.008]. Moreover, the formation of F₂-isoprostanes was studied (determined by measuring the urinary excretion of 8-iso-PGF_{2α,} a non-invasive index of oxidative stress *in vivo*) in the two groups of subjects. As shown in fig. 5F, comparable levels of urinary excretion of F₂-isoprostane were found in carriers of TLR4 polymorphisms and in the wild-type.

### Biosynthesis of prostaglandin E₂ in whole blood stimulated with lipopolysaccharide.

It was determined whether the leukocytes of the carriers of TLR4 polymorphisms show a deficit of recognition of the lipopolysaccharide of *Escherichia coli,* assessing the production of PGE₂ in whole blood stimulated with lipopolysaccharide (cf. Patrignani P, Panara MR, Greco A, et al. Biochemical and pharmacological characterization of the cyclooxygenase activity of human blood prostaglandin endoperoxide synthases. J Pharmacol Exp Ther 1994;271:1705-12). The production of PGE₂ was not significantly different in the carriers of TLR4 polymorphisms and in the wild-type [22 (6-*78) versus* 33 (12-174) ng/ml, respectively, *P*=0.328] (not shown).

### Biosynthesis of prostacyclin and thromboxane in vivo.

The biosynthesis of prostacyclin and of thromboxane *in vivo* was determined by measuring the urinary levels of 2,3-dinor-6-keto-PGF_{1α} and of 11-dehydro- TXB₂, respectively. The urinary excretion of 2,3-dinor-6-keto-PGF_{1α} and 11-dehydro-TXB₂ was significantly lower in carriers of TLR4 polymorphisms than in wild-type subjects [122 (50-577) *versus* 188 (86-436) pg/mg creatinine, respectively, *P*=0.041] (Fig. 6A).

Likewise, the urinary excretion of 11-dehydro-TXB₂ was significantly lower in carriers of *TLR4* polymorphisms than in wild-type subjects[174(63-462) *versus* 540(211-836) pg/mg creatinine, respectively, *P*<0.0001] (Fig. 6B).

In order to exclude a defect of platelet COX-1 in carriers of *TLR4* polymorphisms which could have resulted in a reduced biosynthesis of thromboxane A₂ *in vivo,* we determined the production of TXB₂ in whole blood allowed to clot. The levels of serum TXB₂ did not present a statistically significant difference in the two groups [1.5 (0.6-2.1) *versus* 1.6 (0.8-2.9) ng/10⁸ platelets, respectively, *P*=0.157] (not shown).

The ratio of prostacyclin/thromboxane A₂ biosynthesis was significantly higher in the carriers of *TLR4* polymorphisms than in the wild-type subjects [0.8 (0.3-2.2) *versus* 0.5 (0.11-1.1), respectively, *P*=0.006] (Fig. 6C). Moreover, a statistically significant correlation was found between the urinary excretion of 2,3-dinor-6-keto-PGF_{1α} and 11-dehyro-TXB₂ in carriers of *TLR4* polymorphisms but not in wild-type subjects [rₛ=0.665, n=38, *P*=0.002 *versus* rₛ=-0.07547, n=38, *P*=0.7588, respectively] (Fig. 6D).

### Effects of Rofecoxib (COX-2 inhibitor) on the biosynthesis of prostanoids.

Rofecoxib (VIOXX®), a selective inhibitor of COX-2, was administered in a dose of 25 mg per day, for 5 consecutive days to 6 carriers and non carriers of the genetic polymorphism of TLR4. The effects of Rofecoxib on COX-1 platelet activity were assessed (assessing the production of serum TXB₂ as described in Patrono et al., Thromb Res 1980;17:317-27) and on COAX-2 monocyte activity (measuring the production of PGE₂ in whole blood stimulated with LPS, as described in Patrignani et al., J Pharmacol Exp Ther 1994;271:1705-12). In carriers and non carriers of TLR4 polymorphism, the administration of Rofecoxib does not significantly influence COX-1 platelet activity [-11(from 0 to -57) vs -10(from 0 to -10)% of variation vs pretreatment values, respectively, *P*=0.489], while it causes a profound inhibition of monocyte COX-2 activity *ex vivo* [-94(from -77 to -98) vs -85(from -69 to-97)% inhibition, respectively, *P*=0.589] (Table 8). Rofecoxib causes a selective inhibition of the activity of cyclooxygenase-2 in the two groups of subjects.

**Table 8. Effects of rofecoxib (25 mg per day) administered for 5 consecutive days to 6 carriers of TLR4 polymorphisms and 6 wild-type subjects on the biosynthesis of prostanoids ex vivo and in vivo.**

| | **Pre-drug** | **Rofecoxib** |
|---|---|---|
| **"Wild-type" *TLR4*** | | |
| Serum Thromboxane B₂, ng/ml | 252.5 (176-384) | 233.5 (178-391) |
| Prostaglandin E₂ induced by lipopolysaccharide, ng/ml | 20.3 (14.3-58.1) | 2.3 (0.3-4.2)** |
| 11-dehydro-thromboxane B₂ pg/mg creatinine | 454(227-819) | 345(155-595.6) |
| 2,3 dinor-6 keto-prostaglandin F_{1α}, pg/mg creatinine | 174.5 (77-229) | 56.5 (38-84)* |
| **Carriers of *TLR4* Polymorphisms** | | |
| Serum Thromboxane B₂, ng/ml | 286.5 (125-617) | 225 (94-728) |
| Prostaglandin E₂ induced by lipopolysaccharide, ng/ml | 45.6 (22-67) | 5.3 (1.9-11)** |
| 11-dehydro-thromboxane B₂ pg/mg creatinine | 150 (116-301)^{§} | 329 (158-587)* |
| 2,3 dinor-G-keto-prostaglandin F_{1α}, pg/mg creatinine | 117 (78-285) | 50 (28-93)* |

| | | |
|---|---|---|
| ** *P*<0.01, **P*<0.05, rofecoxib with respect to the values prior to taking the drug; ^{§}*P*<0.01, values prior to taking the drug in carriers of *TLR4* polymorphisms with respect to "wild-type" subjects. | | |

The effects of Rofecoxib on the biosynthesis of systemic prostacyclin and of Thromboxane A₂ were evaluated, As shown in Figure 7 and in Table 8, Rofecoxib causes a similar inhibition of the urinary excretion of 2,3-dinor-6-keto PGF_{1α} [-64(from -42 to -72) vs to -61 (from -35 to -83)%, respectively, *P*=0.818] in the two groups, which was associated with an increased urinary excretion of 11-dehydro-TXB₂ in carriers of TLR4 polymorphisms but not in the "wild-type" subjects [174 (136-354) vs 71 (67-84)% of pre-drug values, respectively, *P*=0.002] (Table 8).

As has already been said, in the absence of treatment with COX-2 inhibitors, the subjects with TLR4 Asp299Gly and Thr399IIe polymorphisms show a decrease of the onset of atherosclerosis and a reduced risk of acute coronary events. From the studies carried out, it was seen that the inhibition by Rofecoxib of the biosynthesis of COX-2-dependent prostacyclin (about 60%) is associated with an increased urinary excretion of 11-dehydro-TXB₂ in carriers of genetic TLR4 polymorphisms but not in "wild-type" subjects, that is in non carriers of polymorphism. The above results lead us to conclude that TLR4 polymorphism increases the consequences of the inhibition of vascular prostacyclin on platelet function by the inhibitors of cyclooxygenase-2. The administration of cyclooxygenase-2 inhibitors in these subjects, carriers of TLR4 polymorphisms, transforms the cardiovascular protection phenotype into an appreciably exposed phenotype which can lead to acceleration of atherogenesis and to an exaggerated thrombotic response following the rupture of the atherosclerotic plaque.

The above results therefore indicate the possibility of identifying and selecting the subjects (that is carriers of TLR4 polymorphisms) who may suffer severe damage from treatment with selective COX-2 inhibitors, with the same initial conditions of the subjects and treatment conditions. So, in these subjects who are carriers of TLR4 polymorphism, the treatment with anti-intlammatory cyclooxygenase-2 inhibitor drugs may provoke serious side effects in the cardiovascular field and thus give sever problems of toxicity.

In consideration of the above, the present invention therefore provides a process for determining the degree of potential toxicity of compounds that are at least inhibitors of cyclooxygenase-2 (COX-2), and possibly of compounds that inhibit both cyclooxygenase-2 and cyclooxygenase-1, which comprises a step of determining the possible presence of genetic TLR4 polymorphism in the subjects to be treated with drugs based on the above-mentioned COX-2 inhibitor compounds.

More in particular said polymorphism is a genetic TLR4 polymorphism (Asp299Gly and/or Thr399IIe) which is determined *"in vitro"* by means of PCR amplification using two pairs of primers for the regions containing the Asp299Gly and Thr399IIe polymorphisms.

The carriers of polymorphism are the subjects for whom the toxicity of said COX-2 inhibitor compounds is greater. This results in the use of compounds that inhibit at least cyclooxygenase-2 (COX-2) as anti-inflammatory drugs only for subjects without genetic TLR4 polymorphism (Asp299Gly and/or Thr399IIe). Said compounds are chosen for example among: Rofecoxib (VIOXX®), Celecoxib (Celebrex®), Valdecoxib (Bextra®) and Lumiracoxib (Prexige®).

## Claims

1. Compounds having the following general formula: 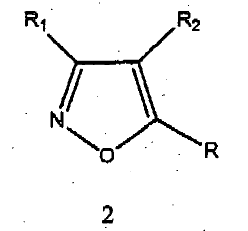 where:
R is a C₁-C₅ alkyl linear, branched, not substituted;
R₁ is 5-chloro-2-furyl,
R₂ is phenyl.

2. Compounds according to claim 1, **characterised in that**
R is a C₁-C₅ alkyl linear.

3. Compounds according to claim 2, **characterised in that** R is chosen as methyl (CH₃) or ethyl (CH₃CH₂).

4. Compounds according to claim 3, **characterised in that** R is chosen as methyl (CH₃).

5. Use of the compounds according to claims 1 to 4, for the manufacture of a medicament as inhibitor of COX-1/COX-2 for the treatment of inflammatory syndromes, in the prevention and treatment of carcinomas, in particular intestinal, ovarian and cutaneous, and in the treatment of pain, in particular deriving from surgery.

6. Use of the compound 3-(5-chlorofuran-2-yl)4-phenyl-5-methylisoxazole (P6), according to claim 5, for the manufacture of a medicament as inhibitor of COX-1 for the treatment of inflammatory syndromes, in the prevention and treatment of carcinomas, in particular intestinal, ovarian and cutaneous, and in the treatment of pain, in particular deriving from surgery.

7. Use of the compounds according to claims 5 or 6, for the preparation of a medicament for the treatment of inflammatory syndromes, in the prevention and treatment of carcinomas, in particular intestinal, ovarian and cutaneous, and in the treatment of pains, in particular deriving from surgery.

8. Compound 3-(5-chlorofuran-2-yl)-4-phenyl-5-methylisoxazole (P6), for the use as a medicament as inhibitor of COX-1 for the treatment of inflammatory syndromes, in the prevention and treatment of carcinomas, in particular intestinal, ovarian and cutaneous, and in the treatment of pain, in particular deriving from surgery.

9. Pharmaceutical composition comprising at least one compound according to claims 1 to 4, in combination with at least a pharmaceutically acceptable carrier, for the treatment of inflammatory syndromes, in the prevention and treatment of carcinomas, in particular intestinal, ovarian and cutaneous, and in the treatment of pain, in particular deriving from surgery.

10. Use of the compounds according to claims 5 or 6, for the preparation of a medicament for the prevention and/or treatment of atherosclerosis.

## Patentansprüche

1. Verbindungen mit der folgenden allgemeinen Formel: 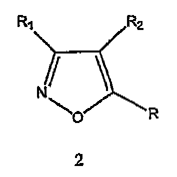 wobei:
R einen unverzweigten oder verzweigten, nicht substituierten C₁-C₅-Alkylrest darstellt;
R₁ einen 5-Chlor-2-Furyl-Rest darstellt;
R₂ einen Phenylrest darstellt.

2. Verbindungen gemäß Anspruch 1, **dadurch gekennzeichnet, dass** R einen unverzweigten C₁-C₅-Alkylrest darstellt.

3. Verbindungen gemäß Anspruch 2, **dadurch gekennzeichnet, dass** R ausgewählt ist aus Methylrest (-CH₃) oder Ethylrest (-CH₂CH₃),

4. Verbindungen gemäß Anspruch 3, **dadurch gekennzeichnet, dass** R einen Methylrest (-CH₃) darstellt.

5. Verwendung von Verbindungen gemäß den Ansprüchen 1 bis 4, zur Herstellung eines Arzneimittels als Hemmer für COX-1/COX-2, zur Behandlung von entzündlichen Syndromen, in der Vorbeugung und Behandlung von Karzinomen, insbesondere intestinale, ovariale und kutane, und zur Behandlung von Schmerz, insbesondere durch Operationen verursachten.

6. Verwendung der Verbindung 3-(5-Chlorfuran-2-yl)-4-Phenyl-5-Methylisoxazol (P6) gemäß Anspruch 5, zur Herstellung eines Arzneimittels als Hemmer für COX-1 zur Behandlung von entzündlichen Syndromen, in der Vorbeugung und Behandlung von Karzinomen, insbesondere intestinale, ovariale und kutane, und zur Behandlung von Schmerz, insbesondere durch Operationen verursachten.

7. Verwendung der Verbindungen gemäß den Ansprüchen 5 oder 6, zur Herstellung eines Arzneimittels zur Behandlung von entzündlichen Syndromen, in der Vorbeugung und Behandlung von Karzinomen, insbesondere intestinale, ovariale und kutane, und zur Behandlung von Schmerz, insbesondere durch Operationen verursachten.

8. Die Verbindung 3-(5-Chlorfuran-2-yl)-4-Phenyl-5-Methylisoxazol (P6), zur Verwendung als Arzneimittel als Hemmer für COX-1 zur Behandlung von entzündlichen Syndromen, in der Vorbeugung und Behandlung von Karzinomen, insbesondere intestinale, ovariale und kutane, und zur Behandlung von Schmerz, insbesondere durch Operationen verursachten.

9. Pharmazeutische Zusammensetzung enthaltend mindestens eine Verbindung gemäß den Ansprüchen 1 bis 4, in Kombination mit zumindest einem pharmazeutisch annehmbaren Träger, zur Behandlung von entzündlichen Syndromen, in der Vorbeugung und Behandlung von Karzinomen, insbesondere intestinale, ovariale und kutane, und zur Behandlung von Schmerz, insbesondere durch Operationen verursachten.

10. Verwendung der Verbindungen gemäß den Ansprüchen 5 oder 6, zur Herstellung eines Arzneimittels zur Vorbeugung und/oder Behandlung von Arteriosklerose.

## Revendications

1. Composés ayant la formule générale suivante : 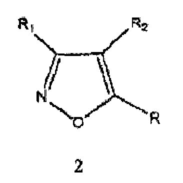 où :
R est un alkyle en C₁ à C₅ linéaire, ramifié, non substitué ;
R₁ est un 5-chloro-2-furyle ;
R₂ est un phényle.

2. Composés selon la revendication 1, **caractérisés en ce que**
R est un alkyle en C₁ à C₅ linéaire.

3. Composés selon la revendication 2, **caractérisés en ce que** R est choisi parmi un méthyle (CH₃) ou un éthyle (CH₃CH₂).

4. Composés selon la revendication 3, **caractérisés en ce que** R est choisi comme un méthyle (CH₃).

5. Utilisation des composés selon les revendications 1 à 4, pour la fabrication d'un médicament inhibiteur de COX-1/COX-2 pour le traitement des syndromes inflammatoires, dans la prévention et le traitement des carcinomes, en particulier intestinaux, ovariens et cutanés, et dans le traitement de la douleur, résultant en particulier d'une opération chirurgicale.

6. Utilisation du composé 3-(5-chlorofuran-2-yl)-4-phényl-5-méthylisoxazole (P6) selon la revendication 5, pour la fabrication d'un médicament inhibiteur de COX-1 pour le traitement des syndromes inflammatoires, dans la prévention et le traitement des carcinomes, en particulier intestinaux, ovariens et cutanés, et dans le traitement de la douleur, résultant en particulier d'une opération chirurgicale.

7. Utilisation des composés selon la revendication 5 ou 6, pour la préparation d'un médicament pour le traitement des syndromes inflammatoires, dans la prévention et le traitement des carcinomes, en particulier intestinaux, ovariens et cutanés, et dans le traitement de la douleur, résultant en particulier d'une opération chirurgicale.

8. Composé 3-(5-chlorofuran-2-yl)-4-phényl-5-méthylisoxazole (P6), pour un usage en tant que médicament inhibiteur de COX-1 pour le traitement des syndromes inflammatoires, dans la prévention et le traitement des carcinomes, en particulier intestinaux, ovariens et cutanés, et dans le traitement de la douleur, résultant en particulier d'une opération chirurgicale.

9. Composition pharmaceutique comprenant au moins un composé selon les revendications 1 à 4, en combinaison avec au moins un support pharmaceutiquement acceptable, pour le traitement des syndromes inflammatoires, dans la prévention et le traitement des carcinomes, en particulier intestinaux, ovariens et cutanés, et dans le traitement de la douleur, résultant en particulier d'une opération chirurgicale.

10. Utilisation des composés selon les revendications 5 ou 6, pour la préparation d'un médicament destiné à la prévention et/ou au traitement de l'athérosclérose.
